# EUROPEAN PATENT APPLICATION

(11) **EP 3 933 030 A1**
(43) Date of publication of application: **05.01.2022**
(21) Application number: 20763093.0
(22) Date of filing: 27.02.2020
(51) Int. Cl.: C12N 1/20, A23C 9/12, A23K 10/16, A23L 29/00, A23L 33/135, A61K 35/74, A61P 1/16, A61P 3/04

(54) **COMPOSITION FOR PREVENTING, ALLEVIATING, OR TREATING OBESITY OR FATTY LIVER DISEASE, COMPRISING WEISSELLA HELLENICA WIKIM0103**

(30) Priority: 28.02.2019 KR 20190024255
(71) Applicant: Korea Food Research Institute, Wanju-gun, Jeollabuk-do 55365 (KR)
(72) Inventor: CHOI, Hak-Jong, Gwangju 62065 (KR); LIM, Seul-Ki, Gwangju 62063 (KR)
(74) Representative: Pons
(86) International application number: PCT/KR2020/002844
(87) International publication number: WO 2020/175939

(57) **Abstract**

The present invention relates to novel *Weissella hellenica* WiKim0103 isolated from Kimchi and a composition comprising the same or its use. The present invention provides a method for prevention, improvement and treatment of inflammation, obesity or fatty liver disease of a subject by administering the novel *Weissella hellenica* WiKim0103 isolated from Kimchi or a composition comprising thereof. The *Weissella hellenica* WiKim0103 according to the present invention shows an inhibitory effect for fatty liver production by inhibiting fat accumulation in liver cells and reducing expression of fatty liver-related genes, and thus it may be variously utilized for uses in prevention, improvement and treatment of inflammation, obesity or fatty liver disease.

<110>     Korea Food Research Institute

<120>     Composition for preventing, improving or treating obesity or
          fatty liver disease comprising the Weissella hellenica WiKim0103

<130>     PCT20-007

<160>     1

<170>     KoPatentIn 3.0

<210>     1
<211>     1444
<212>     DNA
<213>     Unknown

<220>
<223>     Weissella hellenica WiKim0103


<400>     1
gatgaacgct ggcggcgtgc ctaatacatg caagtcgaac gctttgtgct taattgatat        60

gacgagcttg ctctgatttg attttttgat ttcaaagagt ggcgaacggg tgagtaacac        120

gtgggtaacc tacctcttag caggggataa catttggaaa caagtgctaa taccgtataa        180

taccaacaac cgcatggttg ttggttgaaa gatggttctg ctatcactaa gagatggacc        240

cgcggtgcat tagctagttg gtaaggtaat ggcttaccaa ggcaatgatg catagccgag        300

ttgagagact gatcggccac aatgggactg agacacggcc catactccta cgggaggcag        360

cagtagggaa tcttccacaa tgggcgcaag cctgatggag caacgccgcg tgtgtgatga        420

agggtttcgg ctcgtaaaac actgttataa gagaagaacg gcactgagag taactgttca        480

gtgtgtgacg gtatcttacc agaaaggaac ggctaaatac gtgccagcag ccgcggtaat        540

acgtatgttc caagcgttat ccggatttat tgggcgtaaa gcgagcgcag acggttattt        600

aagtctgaag tgaaagccct cagctcaact gaggaattgc tttggaaact ggatgacttg        660

agtgcagtag aggaaagtgg aactccatgt gtagcggtga aatgcgtaga tatatggaag        720

aacaccagtg gcgaaggcgg ctttctggac tgtaactgac gttgaggctc gaaagtgtgg        780

gtagcaaaca ggattagata ccctggtagt ccacaccgta aacgatgagt gctagatgtt        840

cgagggtttc cgcccttgag tgtcgcagct aacgcattaa gcactccgcc tggggagtac        900

gaccgcaagg ttgaaactca aaggaattga cggggacccg cacaagcggt ggagcatgtg        960

gtttaattcg aagcaacgcg aagaacctta ccaggtcttg acatcccttg acaacgctag       1020

aaatagcgcg ttcccttcgg ggacaaggtg acaggtggtg catggttgtc gtcagctcgt       1080

gtcgtgagat gttgggttaa gtcccgcaac gagcgcaacc cttattatta gttgccagca       1140

ttcagttggg cactctagtg agactgccgg tgataaaccg gaggaaggtg gggatgacgt       1200

caaatcatca tgccccttat gacctgggct acacacgtgc tacaatggca tatacaacga       1260

gtcgctaacc cgcgagggta cgctaatctc ttaaagtatg tctcagttcg gattgtaggc 1320

tgcaactcgc ctacatgaag tcggaatcgc tagtaatcgc ggatcagaac gccgcggtga 1380

atacgttccc gggtcttgta cacaccgccc gtcacaccat gagagtttgt aacacccaaa 1440

gccg 1444

## Description

### [TECHNICAL FIELD]

The present application claims the priority based on Korean Patent Application No. 10-2019-0024255 filed on February 28, 2019, and the entire contents disclosed in the description and drawings of the corresponding application are incorporated in the present application. The present invention relates to a novel strain isolated from Kimchi and a composition for preventing, improving or treating inflammation, obesity or fatty liver disease comprising the same.

### [BACKGROUND ART]

Fatty liver is a disease in which normal fat metabolism is not achieved due to excessive intake of fat, increase in accumulation and synthesis in the liver, and decrease in excretion, resulting in accumulation of triglycerides in hepatocytes, and when fat accounts for more than 5% of the total liver weight, it is classified as fatty liver.

In addition, due to the recent westernized high-fat, high-calorie westernized diet and lack of exercise because of the development of civilization, the number of fatty liver patients is increasing rapidly, and the age is also increasing from teenagers to after 60s. When fat is continuously accumulated in the liver, it progresses to fatty hepatitis accompanied by inflammation, and liver cirrhosis may occur due to liver tissue necrosis and fibrosis of the liver because of chronic inflammation. When symptoms worsen further, there is a possibility of developing liver cancer. Therefore, fatty liver is the biggest cause of hepatitis, cirrhosis and liver cancer.

Drugs currently in use on the market include Xenical^{™} (Roche) which has orlistat as a main ingredient, and Reductil^{™} (Abbott) which has sibutramine as a main ingredient, and the like, but show side effects such as diarrhea, abdominal pain, and insomnia, and the like, and as a therapeutic agent of fatty liver, fibrate-based drugs represented by clofibrate, and the like are used in clinical practice, but side effects such as liver dysfunction have been reported.

On the other hand, although various lactic acid bacteria in the following Patent document 1 show the effect of improving fatty liver, there is an urgent need to develop a therapeutic agent for fatty liver which has a fundamental effect of preventing or improving fatty liver and does not exhibit the aforementioned side effects.

Meanwhile, Patent document 1 discloses the effect of improving fatty liver of various lactic acid bacteria such as *Bifidobacterium* sp. and *Lactobacillus* sp. and the like, but a therapeutic agent hat has a fundamental therapeutic effect on fatty liver and does not exhibit the aforementioned side effects has not yet been developed. Therefore, there is an urgent need to develop a therapeutic agent that can fundamentally treat fatty liver and has no side effects.

(Patent document 1) KR No. 10-1937365 (2018.08.08.)

### [DISCLOSURE]

### [TECHNICAL PROBLEM]

The present invention is to provide a novel *Weissella hellenica* sp. lactic acid bacterium with excellent efficacy for prevention or improvement of fatty liver disease.

### [TECHNICAL SOLUTION]

Accordingly, as the result that the present inventors have tried to find a lactic acid bacteria strain which shows an effect for prevention or improvement of fatty liver from traditional fermented foods, they have isolated and identified a novel *Weissella* sp. lactic acid bacteria strain, *Weissella hellenica* WiKim0103, thereby completing the present invention.

One embodiment of the present invention provides a composition for preventing or improving inflammation, obesity or fatty liver disease comprising *Weissella hellenica* WiKim0103 (accession number KCCM12419P), its culture, its lysate or its extract as an active ingredient.

The fatty liver disease may be any one or more selected from the group consisting of simple fatty liver, non-alcoholic steatohepatitis, liver fibrosis and liver cirrhosis.

The composition may be a food composition, a lactic acid bacteria starter composition for food fermentation, a feed or feed additive composition, or a pharmaceutical composition.

The food composition may comprise a health functional food, beverage, bar or fermented oil.

Another embodiment of the present invention provides a use for preventing, improving or treating inflammation, obesity or fatty liver disease of *Weissella hellenica* WiKim0103 (accession number KCCM12419P), its culture, its lysate or its extract.

The fatty liver disease may be any one or more selected from the group consisting of simple fatty liver, non-alcoholic steatohepatitis, liver fibrosis and liver cirrhosis.

It may be used as a food composition, a lactic acid bacteria starter composition for food fermentation, a feed or feed additive composition, or a pharmaceutical composition.

One embodiment of the present invention provides a method for preventing, improving or treating inflammation, obesity or fatty liver disease, comprising administering a composition comprising *Weissella hellenica* WiKim0103 (accession number KCCM12419P), its culture, its lysate or its extract to a subject in need thereof. The subject may be an animal including a human.

A method for preventing, improving or treating inflammation, obesity or fatty liver disease, in which the fatty liver disease comprises any one or more selected from the group consisting of simple fatty liver, non-alcoholic steatohepatitis, liver fibrosis and liver cirrhosis is provided.

The present invention provides *Weissella hellenica* WiKim0103.

The *Weissella hellenica* WiKim0103 of the present invention is a *Weissella hellenica* novel strain derived from Kimchi. Although the *Weissella hellenica* WiKim0103 is isolated and identified from Kimchi in the present invention, but the means of acquisition is not limited thereto.

The lactic acid bacteria strain isolated by examples in the present invention was shown to have the nucleic acid sequence of SEQ ID NO: 1, as the result of 16S rDNA sequencing for identification and classification of microorganisms.

Thus, the microorganism of the present invention which has the 16S rDNA sequence of SEQ ID NO: 1 was named *Weissella hellenica* WiKim0103, and was deposited to Korean Culture Center of Microorganisms on December 14, 2018 (accession number KCCM12419P).

The *Weissella hellenica* WiKim0103 of the present invention is a gram-positive bacterium and a facultative anaerobe which can grow under both the aerobic and anaerobic conditions, and it does not form spores, has no motility and cells are in the form of bacilli.

In the following examples, it was confirmed that the *Weissella hellenica* WiKim0103 of the present invention inhibited expression of SREBP-1c (Sterol regulatory element-binding protein-1c), FAS (fatty acid synthase), SCD (lipogenic-related gene), COX-2 (cyclooxygenase-2), TNF-α (Tumor necrosis factor-alpha) and NF-κ (inflammatory related gene). This confirmed that the *Weissella hellenica* WiKim0103 strain could prevent or improve fatty liver by inhibiting fatty liver production by about 28 to 35%. Therefore, the *Weissella hellenica* WiKim0103 according to the present invention may be variously utilized for uses in prevention, treatment or improvement of fatty liver disease of a human or animal.

In addition, as could be confirmed in the following examples, the composition comprising the *Weissella hellenica* WiKim0103 as an active ingredient was confirmed to inhibit fat accumulation in hepatocytes (HepG2 cells) and inflammation and improve the efficacy of body weight reduction and blood liver function values and reduce expression of factor genes related to fat metabolism, inflammation and liver fibrosis in the liver tissue. Therefore, the composition may be used as an active ingredient of the composition for prevention, treatment or improvement of inflammation, obesity or fatty liver disease which is one or more selected from the group consisting of simple fatty liver, non-alcoholic steatohepatitis, liver fibrosis and liver cirrhosis.

The present invention provides a composition for prevention, improvement or treatment of inflammation, obesity or fatty liver disease comprising *Weissella hellenica* WiKim0103, its culture, its lysate or its extract as an active ingredient.

The *Weissella hellenica* WiKim0103 comprised in the composition according to the present invention may be present as a living cell or dead cell, and it may be also present in a dried or freeze-dried form. The form and formulation method of lactic acid bacteria suitable to be comprised in various compositions are well known to those skilled in the art. For example, *Weissella hellenica* WiKim0103 may be formulated in the form of a culture obtained by culturing in a known liquid medium or solid medium, or a fermented product obtained by culturing the strain and an additional component together, or an extract obtained by extracting the strain with an organic solvent, or a dissolved substance (or lysate) obtained by dissolving the cell membrane of the strain or crushing or treating homogenization, or the like, but not limited thereto.

In one specific example, the composition may be a composition comprising the *Weissella hellenica* WiKim0103 strain present as a living cell or dead cell.

In the present invention, the fatty liver disease may include simple fatty liver, non-alcoholic steatohepatitis, liver fibrosis and liver cirrhosis.

Furthermore, the fatty liver disease may include both alcoholic fatty liver and non-alcoholic fatty liver, and for example, may be non-alcoholic fatty liver induced by high-fat diet. The non-alcoholic fatty liver disease (NAFLD) may include both primary and secondary non-alcoholic fatty liver, and for example, it may be non-alcoholic fatty liver occurred from primary hyperlipidemia, diabetes or obesity.

In one specific example, the composition may be a pharmaceutical composition for preventing or treating inflammation, obesity or fatty liver disease comprising a culture, lysate, fermented product or extract of the *Weissella hellenica* WiKim0103 strain.

When the composition according to the present invention is used as a pharmaceutical composition, the pharmaceutical composition of the present invention may be prepared by using a pharmaceutically suitable and physiologically acceptable adjuvant, and as the adjuvant, an excipient, disintegrating agent, sweetener, binding agent, coating material, inflating agent, lubricant, glydent or flavoring agent, or the like.

The pharmaceutical composition may be preferably formulated for administration, as a pharmaceutical composition by further comprising one or more pharmaceutically acceptable carriers in addition to the active ingredient described above.

For example, for formulation to a form of a tablet or capsule, the active ingredient may be combined with an oral and non-toxic pharmaceutically acceptable inactive carrier such as ethanol, glycerol, water, and the like. In addition, when desired or needed, an appropriate binding agent, a lubricant, a disintegrating agent and a coloring agent may also be comprised as a mixture. The appropriate binding agent is not limited thereto, but includes natural sugars such as starch, gelatin, glucose or beta-lactose, natural and synthetic gum such as corn sweetener, acacia, tragacanth or sodium oleate, sodium stearate, magnesium stearate, sodium benzoate, sodium acetate, sodium chloride, and the like. The disintegrating agent is not limited thereto, but includes starch, methyl cellulose, agar, bentonite, xanthan gum, and the like. In the composition to be prepared as a liquid solution, as an acceptable pharmaceutical carrier, which is suitable for sterile and biocompatible ones, saline solution, sterile water, Ringer's solution, buffer saline solution, albumin injection solution, dextrose solution, maltodextrin solution, glycerol, ethanol and one or more mixed components thereof may be used, and if needed, other common additives such as an antioxidant, buffer solution, bacteriostatic agent, and the like may be added. In addition, it may be formulated as an injection formulation such as an aqueous solution, suspension, emulsion and the like, pill, capsule, granule or tablet, by additionally adding a diluent, a dispersing agent, a surfactant, a binding agent and a lubricant.

Moreover, by an appropriate method of the corresponding field, by using the method described in Remington's Pharmaceutical Science, Mack Publishing Company, Easton PA, it may be preferably formulated according to each disease or component.

In one specific example, the present invention provides a food composition for preventing or improving inflammation, obesity or fatty liver disease comprising *Weissella hellenica* WiKim0103, its culture, its lysate, its fermented product or its extract as an active ingredient. The food composition may include a form of health functional food or beverage, bar or the like.

In the present invention, the food composition comprising the strain as an active ingredient may comprise a beverage such as fermented oil, and the like. Accordingly, the present invention provides a lactic acid bacteria starter for fermentation consisting of *Weissella hellenica* WiKim0103 or its culture.

The food composition according to the present invention may be used as a functional food or added to various kinds of foods as formulated in the same method as the pharmaceutical composition. The food in which the composition of the present invention is added includes for example, beverages, vitamin complexes, health supplement foods, and the like.

The food composition of the present invention may comprise a component commonly added in food preparation, and for example, it includes protein, carbohydrate, fat, nutrients, seasonings, and flavoring agents. The example of the carbohydrate described above is a common sugar such as monosaccharides, for example, glucose, fructose, etc.; disaccharides, for example, maltose, sucrose, oligosaccharide, etc.; and polysaccharides, for example, dextrin, cyclodextrin, etc., and sugar alcohol such as xylitol, sorbitol, erythritol, and the like. As the flavoring agent, a natural flavoring agent [thaumatin, stevia extract (for example, rebaudioside A, glycyrrhizin, etc.)] and a synthetic flavoring agent (saccharin, aspartame, etc.) may be used. For example, when the food composition of the present invention is prepared as drinks and beverages, it may further comprise citric acid, liquid fructose, sugar, glucose, acetic acid, malic acid, fruit juice and various kinds of plant extracts, and the like.

The composition according to the present invention may be used as a feed additive or feed.

When used as a feed additive, the composition may be prepared as a 20 to 90% high concentrate or in a powder or granule form. The feed additive may further comprise any one or one or more of organic acids such as citric acid, fumaric acid, adipic acid, lactic acid, malic acid, etc., or phosphates such as sodium phosphate, potassium phosphate, acidic pyrophosphate, polyphosphate, etc., or natural antioxidants such as polyphenol, catechin, alpha-tocopherol, rosemary extract, vitamin C, green tea extract, licorice extract, chitosan, tannic acid, phytic acid, etc. When used as a feed, the composition may be formulated as a common feed form, and may comprise a common feed component together.

The feed additive and feed may further comprise grains, for example, ground or crushed wheat, oats, barley, corn and rice; plant protein feeds, for example, feeds having rape, bean and sunflower as a main component; animal protein feeds, for example, blood meal, meat meal, bone meal and fish meal; sugars and milk products, for example, dried components consisting of various kinds of powdered milk and whey powder, and the like, and in addition, may further comprise a nutritional supplement, a digestion and absorption enhancer, a growth promoter, and the like.

The feed additive may be administered alone or administered by combining with other feed additives among edible carriers to an animal. In addition, the feed additive may be easily administered to an animal as topdressing or by directly mixing it to animal feed or as an oral formulation separate from feed. When the feed additive is administered separately to animal feed, by combining with a pharmaceutically acceptable edible carrier well known in the corresponding art, it may be prepared as an immediate release or sustained release formulation. This edible carrier may be solid or liquid, for example, corn starch, lactose, sucrose, bean flake, peanut oil, olive oil, sesame oil and propylene glycol. When a solid carrier is used, the feed additive may be a tablet, capsule, powder, troche or lozenge or topdressing in a fine dispersible form. When a liquid carrier is used, the feed additive may be a formulation of gelatin soft capsule, or syrup or suspension, emulsion or solution.

Furthermore, the feed additive and feed may contain an adjuvant, for example, a preservative, stabilizing agent, wetting agent or emulsifier, solution promoter, or the like. The feed additive may be used by immersing, spraying or mixing to add it to animal feed.

The feed or feed additive of the present invention may be applied to numerous animal diets including mammals, poultry and fish.

In the present invention, the animal includes mammals, and it may be used to as mammals, not only a pig, a cow, sheep, a goat, an experimental rodent, and in addition to the experimental rodent, a pet animal (e.g.: dog, cat) and the like, and it may be used to as poultry, a chicken, a turkey, a duck, a goose, a pheasant and a quail, and the like, and it may be used to as fish, a trout, and the like, but not limited thereto.

The amount of the *Weissella hellenica* WiKim0103 strain comprised in the composition according to the present invention may be about 10⁶ to 10¹² cfu/g based on 1 time, and for example, it may be 10⁷ to 10¹¹ cfu/g, 10⁸ to 10¹⁰cfu/g. When the strain is administered, it is preferable to administer it as a living cell, and before intake, it may be administered as a dead or attenuated form. In addition, when prepared by using culture supernatant, and the like, a sterilization process through a heat treatment process may be additionally performed. The strain amount and daily intake level required to have the minimum effect may vary depending on the body or health condition of a taker, but it may be generally about 10⁶ to 10¹²cfu/g, for example, 10⁷ to 10¹¹ cfu/g, 10⁸ to 10¹⁰cfu/g.

Other embodiment of the present invention may be provided as a use for prevention, treatment or improvement of inflammation, obesity or fatty liver disease of a human or animal of *Weissella hellenica* WiKim0103.

Other embodiment of the present invention provides a use for prevention, improvement or treatment of inflammation, obesity or fatty liver disease of *Weissella hellenica* WiKim0103 (accession number KCCM12419P), its culture, its lysate or its extract.

Other embodiment of the present invention provides *Weissella hellenica* WiKim0103 (accession number KCCM12419P), its culture, its lysate or its extract for prevention, improvement or treatment of inflammation, obesity or fatty liver disease.

One embodiment of the present invention provides a method for preventing, improving or treating inflammation, obesity or fatty liver disease, comprising administering a composition comprising WiKim0103 (accession number KCCM12419P), its culture, its lysate or its extract to a subject in need thereof. The subject is an animal including a human, and the example of the animal may be included in the scope of the present invention as long as the aforementioned animals that ingest feed. The fatty liver disease may comprise any one or more selected from the group consisting of simple fatty liver, non-alcoholic steatohepatitis, liver fibrosis and liver cirrhosis.

Advantages and features of the present invention, and methods for achieving them will become apparent with reference to examples described in detail below. However, the present invention is not limited to the examples disclosed below, but will be implemented in a variety of different forms, and is provided only to make the disclosure of the present invention complete, and to completely inform those skilled in the art to which the present invention belongs, the scope of the invention, and the present invention is only defined by the scope of claims.

### [ADVANTAGEOUS EFFECTS]

The *Weissella hellenica* WiKim0103 according to the present invention is a lactic acid bacterium isolated from Kimchi, and shows an inhibitory effect on fatty liver production through inhibition of intracellular fat accumulation and reduction of expression of fatty liver-related genes, and therefore, it may be variously used for uses in prevention, improvement and treatment of inflammation, obesity or fatty liver disease. Furthermore, it may be usefully used as a starter for fermentation.

### [BRIEF DESCRIPTION OF THE DRAWINGS]

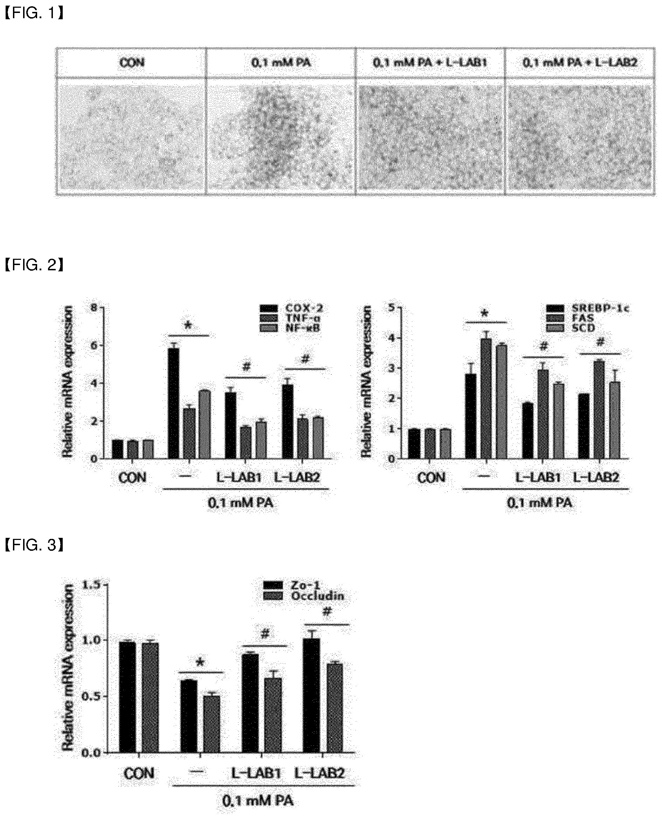

FIG. 1 is a photograph of observing the inhibitory effect of fat accumulation in HepG2 cells according to treatment of the WiKim0103 strain according to the present invention with naked eyes. L-LAB1 represents *W. hellenica* WiKim01013 and L-LAB2 represents *W. koreensis.*
FIG. 2 is a graph of measuring the HepG2 cell-related gene expression level according to treatment of the WiKim0103 strain according to the present invention. L-LAB1 represents *W. hellenica* WiKim01013 and L-LAB2 represents *W. koreensis.* *P<0.05, compare to the CON; #P<0.05, compare to the PA; PA, palmitic acid
FIG. 3 is a graph of measuring the HT-29 cell-related gene expression level according to treatment of the WiKim0103 strain according to the present invention. L-LAB1 represents *W. hellenica* WiKim01013 and L-LAB2 represents *W. koreensis.* *P<0.05, compare to the CON; #P<0.05, compare to the PA; PA, palmitic acid

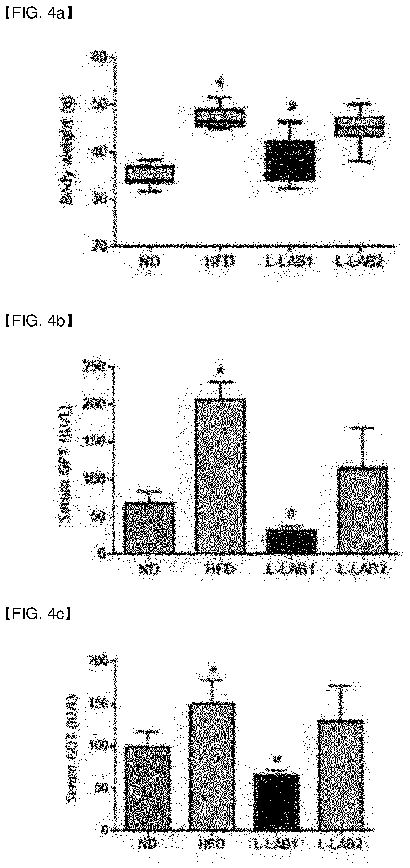

FIG. 4a to FIG. 4c are graphs showing the body weight, blood liver function values (GPT, GOT) of the fatty liver mouse model according to the intake of the WiKim0103 strain according to the present invention. L-LAB1 represents *W. hellenica* WiKim01013 and L-LAB2 represents *W. koreensis.* *P<0.05, compare to the ND; #P<0.05, compare to the HFD.

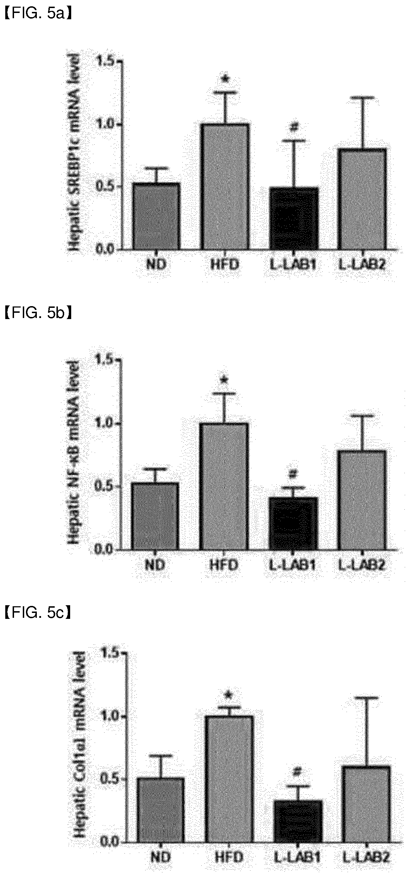

FIG. 5a to FIG. 5c are graphs of measuring the liver tissue-related gene expression level of the fatty liver mouse model according to the intake of the WiKim0103 strain according to the present invention. L-LAB1 represents *W. hellenica* WiKim01013 and L-LAB2 represents *W. koreensis.* *P<0.05, compare to the ND; #P<0.05, compare to the HFD.

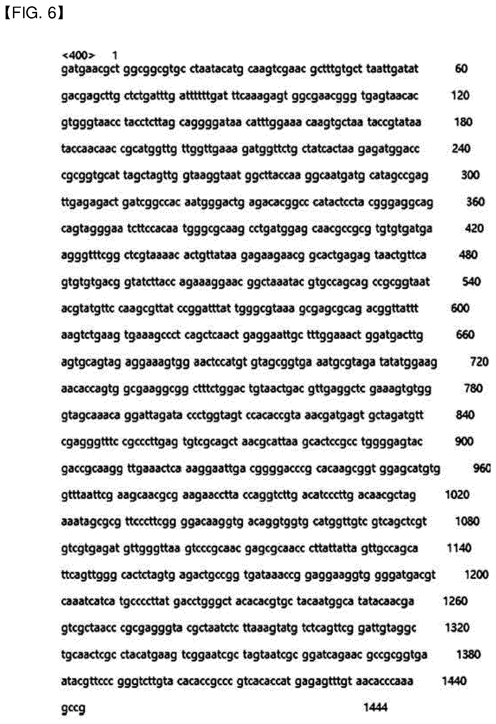

FIG. 6 shows SEQ ID NO: 1 of the present application.

### [MODE FOR INVENTION]

Hereinafter, the present invention will be described in detail by examples. The following examples are intended to illustrate the present invention only, but the scope of the present invention is not limited by the following examples.

### Example 1: Identification of Weissella hellenica WiKim0103

A bacterial single colony obtained by applying an undiluted solution of Kimchi extract on an MRS medium was collected with a loop and cultured in an MRS broth. DNA extraction was performed by using QIAamp DNA Mini Kit (QIAgen, Germany). The extracted DNA was confirmed by using 1 % agarose gel, and to amplify a 16S rDNA gene, Polymerase Chain Reaction (PCR) was processed by using extracted genomic DNA as a template, and 30 cycles were performed under PCR conditions of denaturation at 95°C for 1 minute, annealing at 45°C for 1 minute and extension at 72°C for 1 minute and 30 seconds. For the obtained PCR products, the sequence was analyzed by requesting to Macrogen (Seoul, Korea). Identification of bacteria was performed by inductive analysis of Basic Local Alignment Search Tool (BLAST) search engine of National Center for Biotechnology Information (NCBI, www.ncbi.nlm.nih.gov) for 16S rDNA sequence.

The strain isolated by the example of the present invention was shown to have the nucleic acid sequence of SEQ ID NO: 1, as the result of 16S rDNA sequence analysis for identification of microorganisms.

Accordingly, the microorganism of the present invention was named *Weissella hellenica* WiKim0103, and deposited to Korean Culture Center of Microorganisms on December 14, 2018 (KCCM12419P).

### Example 2: Confirmation of inhibitory efficacy of fat accumulation and inflammation in hepatocytes (HepG2 cells) of Weissella hellenica WiKim0103

An in vitro experimental method was devised using a transwell to mimic a human body gut-liver axis.

The *Weissella hellenica* WiKim0103 lactic acid bacterium was cultured in an MRS medium at 30°C for 24 hours. At the end of the culture, to recover microbial cells, it was prepared by performing centrifugation at 6,000rpm for 5 minutes and rinsing with PBS to remove all remaining medium components.

To culture enterocytes (HT-29 cells) and hepatocytes (HepG2 cells), RPMI-1640 medium in which penicillin/streptomycin and 10% fetal bovine serum were added was used, and co-culture was prepared by using a 6-well plate transwell. Before co-culture, enterocytes were aliquoted in a transwell membrane, and hepatocytes were aliquoted in the 6-well plate, and they were prepared by culturing under the conditions of 37°C, 5% CO₂, respectively.

When the enterocytes (HT-29 cells) and hepatocytes (HepG2 cells) grew about 80% in a culture dish, the culture solution was replaced with an FBS free medium and they were cultured for 16 hours. After culturing, prepared lactic acid bacteria 1X10⁷ CFU were treated to the enterocytes, and co-culture of lactic acid bacteria and enterocytes and hepatocytes was performed by assembling a transwell, and fat production in hepatocytes was induced by treating 0.1 mM palmitic acid for 48 hours. After culturing with 0.1 mM palmitic acid for 48 hours, the enterocytes and hepatocytes were recovered, respectively. Fat production in the hepatocytes was confirmed by oil red-O staining, and expression of fat metabolism and inflammation-related genes, and the permeability of the enterocytes was confirmed by expression of tight junction-related genes in the enterocytes.

### 1) Confirmation of fat accumulation bv Oil red-O stain

The culture solution was removed and they were washed with PBS, and then fixed with 10% formaldehyde for 10 minutes. 10% formaldehyde was removed and saturated formaldehyde was added again to fix cells over 1 hour. Then, 60% isopropanol was added and removed immediately, and then washed with distilled water, and fat globules were stained with oil red-O solution for 30 minutes and then washed with distilled water. The fat accumulated by oil red-O solution represents red (FIG. 1).

As a result, it could be confirmed that fat accumulation was reduced in the hepatocytes (HepG2 cells) in which 0.1 mM palmitic acid and *Weissella hellenica* WiKim0103 were co-cultured than the hepatocytes (control) in which 0.1 mM palmitic acid was treated.

### 2) Confirmation of hepatocytes (HepG2 cells) expression level of fat differentiation and inflammation-related genes

To find out the expression level of fat metabolism and inflammation-related genes in hepatocytes according to the treatment of the *Weissella hellenica* WiKim0103 strain, quantitative real time polymerase chain reaction (qPCR) was conducted.

As a result, as shown in FIG. 2, it could be confirmed that the gene expression of fat metabolism-related factors, SREBP-1c (Sterol regulatory element-binding protein-Ic), fatty acid synthase (FAS), and unsaturated fatty acid biosynthesis enzyme (Stearoyl-CoA desaturase, SCD), and inflammation-related factors, COX-2 (Cyclooxygenase-2), TNF-α (Tumor necrosis factor-alpha), and NF-κB (Nuclear factor-kappa B) was significantly reduced, in the hepatocytes (HepG2 cells) in which 0.1 mM palmitic acid and Weissella hellenica WiKim0103 were co-cultured than the hepatocytes (control) in which 0.1 mM palmitic acid was treated.

### 3) Confirmation of expression level of tight junction-related genes in enterocytes (HT-29 cells)

To find out the expression level of tight junction-related genes in the enterocytes according to the treatment of the *Weissella hellenica* WiKim0103 strain, quantitative real time polymerase chain reaction (qPCR) was conducted.

As a result, as shown in FIG. 3, it could be confirmed that the expression of tight junction-related ZO-I (Zonula occludens-1) and Occludin genes was significantly increased in the enterocytes in which 0.1 mM palmitic acid and *Weissella hellenica* WiKim0103 were co-cultured than the enterocytes (control) in which 0.1 mM palmitic acid was treated.

### Example 3: Confirmation of body weight reduction efficacy and improvement of liver function values in blood

Five-week-old male mice (C57BL/6J) were adapted by chow diet (CD; Purina, Korea) in the breeding room environment under the conditions of temperature 20±2°C, humidity 50±5% and light-dark cycle 12 hours unit for 1 week. The experimental animals were fed a normal diet (D12450B, Research Diets, New Brunswick, NJ) or high-fat diet (D12451, Research Diets, New Brunswick, NJ) in which 45% of total calories were fat, and were adapted to the diet for 1 week, and then the experiment was performed. The experimental groups consisted of 4 groups in total, which were the normal diet intake group (ND), high-fat diet intake group (HFD), intake group receiving daily oral administration of *Weissella hellenica* WiKim0103 at a concentration of 2 X 10⁹ CFU/200µl with the high-fat diet (HFD+L-LAB1), and intake group receiving daily oral administration of *Weissella koreensis* at a concentration of 2 X 10⁹ CFU/200µl with the high-fat diet ((HFD+L-LAB2), and fatty liver was induced through diet for 20 weeks. The normal diet intake group (ND) and high-fat diet intake group (HFD) were orally administered with the same amount of phosphate buffered saline (PBS) daily. In 20 weeks after performing the experiment, the body weight of each experimental group was measured (FIG. 4a). In addition, to measure the liver function values (serum GOT, serum GPT) in blood of the experimental groups, they were fasted 16 hours before the end of the experiment, and blood was collected from the abdominal vena cava, and serum was separated and used in the experiment. The GOT and GPT in blood were measured by using an automatic biochemical measuring device (FUJI DRI-CHEM 7000i, Fujifilm, Tokyo, Japan), and in FIG 4b and FIG 4c, GLUTAMATE-OXALOACETATE TRANSAMINASE (GOT) and GLUTAMATE-PYRUVATE TRANSAMINASE (GPT) in blood were shown as graphs.

As a result, as shown in FIG. 4a to FIG. 4c, it could be confirmed that the body weight and liver function values in blood were significantly reduced when the high-fat diet and *Weissella hellenica* WiKim0103 were ingested together (HFD+L-LAB1), compared to the high-fat diet intake mice (HFD).

### Example 4: Confirmation of expression level of fat metabolism, inflammation and liver fibrosis-related genes in liver tissue

To find out the expression level of fat metabolism, inflammation and liver fibrosis-related genes in the liver tissue according to the intake of the *Weissella hellenica* WiKim0103 strain, using the liver tissue extracted after the end of experiment, qPCR (quantitative real time polymerase chain reaction) was conducted.

As a result, as shown in FIG. 5a to FIG. 5c, it could be confirmed that the expression of genes of the fat metabolism-related factor, SREBP-Ic (Sterol regulatory element-binding protein-Ic), inflammation-related factor, NF-κB (Nuclear factor-kappa B), and liver fibrosis-related factor, Col1α1 (Collagen type I alpha 1) was reduced, according to the intake of the *Weissella hellenica* WiKim0103 strain.

### [INDUSTRIAL AVAILABILITY]

The present invention provides a novel strain. In addition, the strain of the present invention may be used as a food, feed or feed additive, pharmaceutical composition for preventing, improving or treating inflammation, obesity or fatty liver disease, or the like.

### [Accession number]

Depository authority name : Korean Culture Center of Microorganisms (Overseas)
Accession number: KCCM12419P
Accession date : 20181214

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Korea Food research Institute
245, Nongsaengmyeong-ro,
Iseo-myeon, Wanju-gun,
Jeollabuk-do, 55365,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I . IDENTIFICATION OF THE MICROORGANISM
Identification reference given by the
DEPOSITOR:
Weissella hellenica WiKim0103 | Accession number given by the
INTERNATIONAL DEPOSITARY AUTHORITY:
KCCM12419P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 14. 2018 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms
Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):
Date: December. 14. 2018.

¹ Where Rule 6.4(d) applies, such date is the date on which the status of international depositary authority was
acquired.

Form BP/4 (sole page)

특허절차상 미생물 기탁의
국제적 승인에 관한 부다페스트 조약

국제양식

하기 국제 기탁 기관에 의해 규칙 7.1에 따라 발행된
원기탁에 대한 수탁증

기탁자 우편번호 55365, 대한민국 전라북도
완주군 이서면 농생명로 245
한국식품연구원

I. 미생물의 표시
기탁자가 부여한 식별을 위한 표
시:
와이셀라 벨레니카
WiKim0103 | 국제 기탁기관이 부여한 수탁번호:
KCCM12419P
II. 과학적 성질 및/또는 분류학상의 위치
I 란에 지정된 미생물은 다음 사항을 기재한 문서가 첨부되어 있다.
□ 과학적 성질
□ 분류학상의 위치
(해당되는 곳에 십자 표시)
III. 수령 및 수탁
본 국제 기탁 기관은 2018년 12월 14일 (원기탁일)에 수령한 I 란의 미생물
을 수탁하였다.
IV. 전환요구의 수령
I 란의 미생물은 본 국제 기탁 기관에 의해 ____________ (원기탁일)에 수탁
하였고,
오리지날 기탁을 부다페스트 조약에 부합하는 기탁으로 전환하고자 하는 요
청을 ___________ (전환요구의 수령일)에 받았다.
V. 국제 기탁 기관
명칭 : 한국미생물보존센터

주소 : 서울시 서대문구 홍제내2가길
45 유림빌딩, 우편번호 03641 | 국제기탁기관을 대표하는 권한을 갖
는 자 또는 공인된 임원의 서명

서명일 : 2018. 12. 14.

## Claims

1. *Weissella hellenica* WiKim0103 with accession number KCCM12419P.

2. A composition for preventing or improving inflammation, obesity or fatty liver disease comprising *Weissella hellenica* WiKim0103 (accession number KCCM12419P), its culture, its lysate or its extract as an active ingredient.

3. The composition according to claim 2,
wherein the fatty liver disease comprises any one or more selected from the group consisting of simple fatty liver, non-alcoholic steatohepatitis, liver fibrosis and liver cirrhosis.

4. The composition according to claim 2,
wherein the composition is a food composition, a lactic acid bacteria starter composition for food fermentation, a feed or feed additive composition, or a pharmaceutical composition.

5. The composition according to claim 4,
wherein the food composition is a health functional food, beverage, bar or fermented oil.

6. A use for preventing, improving or treating inflammation, obesity or fatty liver disease of *Weissella hellenica* WiKim0103 (accession number KCCM12419P), its culture, its lysate or its extract.

7. The use according to claim 6,
wherein the fatty liver disease comprises any one or more selected from the group consisting of simple fatty liver, non-alcoholic steatohepatitis, liver fibrosis and liver cirrhosis.

8. The use according to claim 6,
used as a food composition, a lactic acid bacteria starter composition for food fermentation, a feed or feed additive composition, or a pharmaceutical composition.

9. The use according to claim 8,
wherein the food composition is a health functional food, beverage, bar or fermented oil.

10. A method for preventing, improving or treating inflammation, obesity or fatty liver disease, comprising administering a composition comprising *Weissella hellenica* WiKim0103 (accession number KCCM12419P), its culture, its lysate or its extract to a subject in need thereof.

11. The method for preventing, improving or treating inflammation, obesity or fatty liver disease according to claim 10,
wherein the subject is an animal including a human.

12. The method for preventing, improving or treating inflammation, obesity or fatty liver disease according to claim 10,
wherein the fatty liver disease comprises any one or more selected from the group consisting of simple fatty liver, non-alcoholic steatohepatitis, liver fibrosis and liver cirrhosis.
